# EUROPÄISCHE PATENTSCHRIFT

(11) **EP 1 380 208 B1**
(45) Veröffentlichungstag und Bekanntmachung des Hinweises auf die Patenterteilung: **29.06.2005**
(21) Anmeldenummer: 03009652.3
(22) Anmeldetag: 30.04.2003
(51) Int. Cl.: A01N 31/02, A01N 31/08, A61K 31/22, A61K 31/05, A61P 33/00

(54) **Desinfektionsmittel mit antiparasitärer Wirkung**
Disinfectant agent with antiparasitic activity
Agent desinfectant à activité antiparasitaire

(30) Priorität: 22.05.2002 DE 10222455
(43) Veröffentlichungstag der Anmeldung: 14.01.2004
(73) Patentinhaber: Ewabo Chemikalien GmbH, 49835 Wietmarschen (DE)
(72) Erfinder: Briesemann, Günter, 49835 Wietmarschen (DE); Beckmann, Guido, 48431 Rheine (DE)
(74) Vertreter: COHAUSZ & FLORACK

(56) Entgegenhaltungen:
- EP-A- 0 339 449
- EP-B- 0 683 628
- DE-A- 19 818 849
- US-A- 5 661 170

## Beschreibung

Die Erfindung betrifft die Verwendung eines Desinfektionsmittels mit antiparasitärer Wirkung, das einen Desinfektionswirkstoff und eine keratolytisch wirkende Verbindung enthält.

Parasitäre Erkrankungen von Zuchttieren stellen bei der Intensivtierhaltung eine erhebliche Gefahr für den Zuchtbetrieb dar. Vor allem der Befall mit pathogenen Einzellern und Würmern führt Jahr für Jahr weltweit zu erheblichen Verlusten in landwirtschaftlichen Betrieben. Weit verbreitet ist der Befall mit Spulwürmern und Kokzidien. Das klinische Spektrum dieser parasitären Erkrankungen reicht vom subklinischen Verlauf ohne spezifische Symptome bis zu Todesfällen vor allem bei Jungtieren.

Der Befall mit Spulwürmern ist die wichtigste Wurmerkrankung des Schweins. Mit Spulwürmern befallene Schweine leiden unter stark verminderter Gewichtszunahme.

Bei Wurmbefall gehört eine regelmäßige Entwurmung zu den wichtigen Maßnahmen zur Heilung und Gesunderhaltung. Um zu vermeiden, dass sich die Tiere erneut durch die Umgebung infizieren, müssen spezielle gegen Spulwurmeier wirkende Desinfektionsmittel eingesetzt werden. Ascaris suis, der Spulwurm des Schweines parasitiert im Dünndarm, wo der weibliche Wurm täglich etwa 100.000 Eier, unter besonderen Bedingungen auch bis zu 1.000.000 Eiern, produziert. Die Eier besitzen eine widerstandsfähige Schale und sind Umwelteinflüssen gegenüber sehr stabil, so dass sie noch nach fünf Jahren infektiös sein können. Spezialdesinfektionsmittel mit Wirksamkeit gegen Wurmeier enthalten als Wirkstoffe entweder Kresole oder Kombinationen aus Phenolen, Schwefelkohlenstoff und Chloroform. Jedoch können die Wirkstoffe die widerstandsfähige Schale der Eier nur schwer durchdringen.

Daher enthalten herkömmliche Desinfektionsmittel neben den desinfizierenden Wirkstoffen noch organische Lösungsmittel, chlorierte Kohlenwasserstoffe oder Schwefelwasserstoff, um das Eindringen der desinfizierenden Wirkstoffe in das Innere der Wurmeier zu unterstützen. Lösungsmittel sind jedoch aus ökologischer und toxikologischer Sicht als sehr bedenklich einzustufen.

Die US 5 661 170 betrifft antimikrobielle Zusammensetzungen zur Behandlung des menschlichen oder tierischen Körpers, die Allantoin als keratolytisch wirkendes Mittel enthalten. Darüber hinaus sind Methyl- oder Propylparaben als Konservierungsmittel enthalten.

Die DE 198 18 849 A1 offenbart einen Ester der Glykolsäure zur therapeutischen oder kosmetischen Behandlung der Haut, wobei festgestellt wird, dass Glykolsäureesther besser in tiefere Schichten der Haut eindringen als Glykolsäure.

Die EP 0 339 449 betrifft ein Parasiten abtötendes Desinfektionsmittel, das neben einem Desinfektionswirkstoff ein Sulfonatsalz eines Ester einer ungesättigten C₁₆-C₂₂-Fettsäure mit einem geradkettigen oder verzweigten aliphatischen C₁-C₆-Alkohol (UFSE-Sulfonat) enthält. Das Sulfonat erhöht die Permeabilität der Hülle der Dauerformen und ermöglicht so ein besseres Eindringen des Desinfektionswirkstoffs.

Die EP 0 683 628 B1 beschreibt ein Desinfektionsmittel, das ein oder mehrere Phenole, Ethylenglykoldialkylether, Emulgatoren und eine organische Säure wie Salicylsäure enthält. Es wurde festgestellt, dass keratolytisch wirkende organische Säuren im Gemisch mit Phenolen durch die chitinähnliche mittlere Schicht von Askarideneiern wandern können. Das Desinfektionsmittel zeigte in 2%iger Lösung und angewendet über einen Zeitraum von zwei Stunden eine günstige Wirkung. Um eine 100%ige Abtötung der Eier zu erreichen, sind jedoch längere Behandlungszeiten erforderlich.

Der Erfindung lag somit die Aufgabe zugrunde, ein Mittel mit verbesserter Wirkung zur Bekämpfung eines breiten Spektrums von Parasiten bereitzustellen.

Gelöst wird diese Aufgabe durch die Verwendung eines Desinfektionsmittel mit antiparasitärer Wirkung, das mindestens einen Desinfektionswirkstoff und mindestens eine keratolytisch wirkende Verbindung enthält, zur nichtmedizinischen Bekämpfung von Askariden, Kokzidien sowie deren invasionsfähigen Dauerformen, Protozoen, Zecken, Läuse, Kratzmilben und Mikroorganismen,
wobei die keratolytisch wirkende Verbindung ein Ester einer organischen oder anorganischen keratolytisch wirkenden Säure und eines ein- oder mehrwertigen Alkohols mit 1 bis 6 Kohlenstoffatomen ist.

Überraschend wurde festgestellt, dass mit den erfindungsgemäßen verwendeten Desinfektionsmitteln, die im Unterschied zu den Desinfektionsmitteln der EP 0 683 628 B1 einen keratolytisch aktiven Ester anstatt einer freien Säure enthalten, in einer kürzeren Behandlungszeit bei geringeren Konzentrationen des Mittels eine vollständige Abtötung der Eier und Oozysten erzielt wird.

Das erfindungsgemäße verwendete Desinfektionmittel weist eine sehr gute keratolytische Tiefenwirkung auf. Keratolytische Tiefenwirkung bedeutet, dass der Desinfektionswirkstoff durch kreatinhaltige Schichten wie Parasitenmembranen dringt.

Unter antiparasitärer Wirkung im Sinne der Erfindung wird die Fähigkeit einer irreversiblen Schädigung einer Vielzahl von Parasiten und deren Dauerformen verstanden. Unter Parasiten im Sinne der Erfindung sind beispielsweise alle belebten tierischen Krankheitserreger wie Protozoen, Würmer, Zecken, Läuse, Kratzmilben und Mikroorganismen zu verstehen.

Erfindungsgemäß geeignete Ester sind Ester anorganischer oder organischer keratolytisch wirkender Säuren. Geeignete anorganische Säuren sind beispielsweise Chromsäure und schwefelige Säure. Geeignete organische Säuren sind Carbonsäuren mit 1 bis 10 Kohlenstoffatomen, beispielsweise aliphatische Mono-, Di- und Tricarbonsäuren, insbesondere auch deren hydroxylierte und/oder halogenierte Derivate und heterocyclische Carbonsäurenaromatische Carbonsäuren, insbesondere auch deren hydroxylierte und/oder halogenierte Derivate und/oder Mono-, Di- und Tricarbonsäuren.

Geeignete Säureester basieren deshalb beispielsweise auf Ameisensäure, Essigsäure, Trichloressigsäure, Propionsäuren, Oxalsäure, Malonsäure, Bernsteinsäure, Glykolsäure, Thioglykolsäure, Milchsäure, Thiomilchsäure, Äpfelsäure, Weinsäure, Citronensäure, Salicylsäure und Gentisinsäure, Dipicolinsäure, Picolinsäure, Nicotinsäure, Isonicotinsäure, deren Ammonium-, Alkali- und Erdalkalisalze sowie deren Gemische.

Erfindungsgemäß geeignete Estergruppen sind ein- oder mehrwertige Alkohole mit 1 bis 5 oder 6, vorzugsweise 1 bis 3, Kohlenstoffatomen. Als Estergruppen geeignete einwertige Alkohole sind beispielsweise Methanol, Ethanol, Propanol und Isopropanol. Geeignete mehrwertige Alkohole sind beispielsweise Glykol und Glycerol, wobei eine oder zwei der Hydroxylgruppen des mehrwertigen Alkohols verestert sein können. Besonders bevorzugt werden Glycerinester, die ein besonders gutes Durchdringen des Desinfektionsmittels in das Innere des Parasiten oder dessen Dauerform ermöglichen.

Die erfindungsgemäß verwendeten Ester können in dem Desinfektionsmittel vorzugsweise in einem Anteil von 10 bis 35 Gew.%, besonders bevorzugt 15 bis 25 Gew.%, bezogen auf die Masse des Mittels, enthalten sein.

Das erfindungsgemäße verwendete Desinfektionsmittel enthält mindestens einen Desinfektionswirkstoff. Erfindungsgemäß einsetzbare Desinfektionswirkstoffe können bekannte Desinfektionswirkstoffe sein, beispielsweise phenolische Verbindungen. Geeignete phenolische Wirkstoffe sind Phenol, substituierte Phenole, Kresole und halogenierte Kresole, 2-Methylphenol, 3-Methylphenol, 4-Methylphenol, 4-Ethylphenol, 2,4-Dimethylphenol, 2,5-Dimethylphenol, 3,4-Dimethylphenol, 2,6-Dimethylphenol, 4-n-Propylphenol, 4-n-Butylphenol, 4-n-Amylphenol, 4-n-Hexylphenol, Thymol, o-Cyclohexyl-p-chlorphenol, o-n-Amyl-p-chlorphenol, o-n-Hexyl-p-chlorphenol, p-Chlor-m-kresol, 4-tert.-Butyl-2,6-dichlorphenol, 6-tert.-Butyl-4-chlor-m-kresol, 4-Ethyl-4chlorphenol, 4-Chlor-3,5-xylenol, 2,4-Dichlor-3,5-xylenol, p-Phenylphenol, o-Phenylphenol, 2-Benzylphenol, p-Chlor-o-phenylphenol, Benzyl-4-chlor-m-kresol und 4-Chlorbenzyldichlor-m-kresol. Besonders bevorzugt wird p-Chlor-m-kresol eingesetzt.

Die erfindungsgemäß verwendeten Desinfektionswirkstoffe können in dem Desinfektionsmittel vorzugsweise in einem Anteil von 10 bis 35 Gew.%, besonders bevorzugt 15 bis 25 Gew.%, bezogen auf die Masse des Mittels, enthalten sein.

Das erfindungsgemäße verwendete Desinfektionsmittel kann eine alkalisch wirkende Lauge enthalten. Eine geeignete Lauge ist beispielsweise Natronlauge. Die Lauge kann in einer Konzentration von 1 bis 15 Gew.%, bezogen auf das Gewicht des Desinfektionsmittels, vorhanden sein.

Im erfindungsgemäßen verwendeten Desinfektionsmittel kann gemäß einer weiteren bevorzugten Ausführungsform eine anorganische Säure enthalten sein. Eine geeignete anorganische Säure ist beispielsweise Salzsäure. Die anorganische Säure kann in einer Konzentration von 0,1 bis 10 Gew.%, bezogen auf das Gewicht des Desinfektionsmittels, vorliegen. Im sauren Bereich ist die keratolytische Wirkung zwar geringfügig schlechter als im basischen Bereich; die desinfizierende Wirkung ist jedoch höher.

Der pH-Wert des erfindungsgemäßen verwendeten Desinfektionsmittels im Konzentrat kann in einem Bereich von 3 bis 10, vorzugsweise 4 bis 9, liegen.

Die erfindungsgemäßen verwendeten Desinfektionsmittel können mindestens ein anionisches und/oder nichtionisches Tensid enthalten. Geeignete nichtionische Tenside sind beispielsweise höhere Fettalkohole, Partialfettsäureester mehrwertiger Alkohole, Polyethylenglykolfettsäureester, Polyethylenglykolfettsäureether, Polyethylenglykolsorbitanfettsäureester, Alkoholammoniumfettalkoholsulfate.

Erfindungsgemäß geeignete anionische Tenside sind beispielsweise Alkaliseifen, Aminseifen, Alkylsulfate, Alkylsulfonate (C₁₀₋₁₃)-n-Alkylacrylsulfonate wie (C₁₀₋₁₃)-Alkylbenzolsulfonate.

Die Tenside können vorzugsweise in einer Konzentration von 10 bis 35 Gew.%, besonders bevorzugt 15 bis 30 Gew.%, bezogen auf die Masse des Desinfektionsmittels, enthalten sein. Die Tenside dienen der besseren Verteilung der Wirkstoffe auf den zu desinfizierenden Flächen und, falls erforderlich, der Stabilisierung der erfindungsgemäßen verwendeten Zubereitungen.

Die erfindungsgemäßen verwendeten Desinfektionsmittel können weitere Hilfsstoffe enthalten. Solche Hilfsstoffe sind beispielsweise Lösungsmittel wie kurzkettige ein oder mehrwertige Alkohole, beispielsweise Methanol, Ethanol, Isopropanol, Glykol und/oder deren Ether wie Ethylenglykoldialkylether. Vorzugsweise wird Ethanol als Lösungsmittel verwendet. Lösungsmittel können in einer Konzentration von 10 bis 30 Gew.%, vorzugsweise etwa 20 Gew.%, bezogen auf die Masse des Desinfektionsmittels, enthalten sein. Die Zugabe eines Lösungsmittel erleichtert die Herstellung des Desinfektionsmittels.

Die erfindungsgemäß verwendeten Desinfektionmittel eignen sich zur Bekämpfung von Askariden, Kokzidien sowie deren invasionsfähigen Dauerformen wie Eiern und Oozysten.

### Beispiel 1 (Vergleichsversuch)

In dem folgenden Vergleichsversuch wird die antiparasitäre Wirkung der erfindungsgemäßen keratolytisch wirkenden Ester verglichen mit den aus der EP 0 683 628 B1 bekannten keratolytisch wirksamen Säuren. Die Rezepturen 1 und 2 betreffen erfindungsgemäße Ester, Rezeptur 3 ist ein Vergleichsbeispiel. Die Rezepturen sind in der folgenden Tabelle aufgezeigt.

| Rezeptur | 1 | 2 | 3 |
|---|---|---|---|
| Kresol | 25% | 25% | 25% |
| Thioglykolsäureglycerinester | 25% | - | - |
| 3-Mercaptopropionsäureglycerinester | - | 25% | - |
| Thioglykolsäure | - | - | 25% |
| Emulgator | 25% | 25% | 25% |
| Ethanol | 25% | 25% | 25% |

Bei den Zusammensetzungen gemäß Rezeptur 1 und 2 wurde der pH-Wert auf 1,7 bis 2 mit HCl eingestellt. Diesen pH-Wert weist auch die Zusammensetzung gemäß Rezeptur 3 auf.

Zur Feststellung der Wirksamkeit der in der Tabelle gezeigten Zusammensetzungen wurde zunächst von jeder Zusammensetzung eine 8%ige Lösung hergestellt und diese 1:1 mit einer Kokzidiensuspension vermischt, so daß sich eine 4%ige Testlösung ergab. Das Gemisch wurde 2 Stunden bei 25°C belassen und anschließend in destilliertes Wasser überführt. Durch die Verdünnung mit destilliertem Wasser wird die Reaktion unterbrochen. Anschließend wird abdestilliert und zweimal bei 2500 U/min 10 Minuten lang zentrifugiert. Vom Sediment wurden jeweils 1 ml entnommen und mit 9 ml einer 0,9%igen NaCl-Lösung gemischt.

Die Kontrollprobe ist ein 1:1-Gemisch aus Kokzidiensuspension und Aqua dest.

Anschließend erfolgte die Auszählung in sogenannten Masterkammern. Dazu wurden je Probe 10 Tüpfel a 50 µl ausgezählt. Festgestellt wurden dabei die noch vorhandenen zur Embryonierung fähigen Oocysten. Die Ergebnisse sind in der Tabelle kurz dargestellt.

| | Kontrolllösung | Rezeptur 1 | Rezeptur 2 | Rezeptur 3 |
|---|---|---|---|---|
| Mittlere Anzahl Oozysten | 20 | 1/6 | 0,5 | 6 |

## Patentansprüche

1. Verwendung eines Desinfektionsmittels mit antiparasitärer Wirkung, das mindestens einen Desinfektionswirkstoff und mindestens eine keratolytisch wirkende Verbindung enthält, zur nichtmedizinischen Bekämpfung von Askariden, Kokzidien sowie deren invasionsfähigen Dauerformen, Protozoen, Zecken, Läuse, Kratzmilben und Mikroorganismen, wobei die keratolytisch wirkende Verbindung ein Ester einer organischen oder anorganischen keratolytisch wirkenden Säure und eines ein- oder mehrwertigen Alkohols mit 1 bis 6 Kohlenstoffatomen ist.

2. Verwendung nach Anspruch 1, **dadurch gekennzeichnet, dass** die organische Säure Thioglykolsäure ist.

3. Verwendung nach Anspruch 1 oder 2, **dadurch gekennzeichnet, dass** der Ester ein Glycerinester ist.

4. Verwendung nach einem der Ansprüche 1 bis 3, **dadurch gekennzeichnet, dass** der Ester in einem Anteil von 10 bis 30 Gew.% vorliegt.

5. Verwendung nach einem der Ansprüche 1 bis 4, **dadurch gekennzeichnet, dass** die Desinfektionswirkstoffe phenolische Desinfektionswirkstoffe, insbesondere Chlorkresole sind.

6. Verwendung nach.Anspruch 5, **dadurch gekennzeichnet, dass** das Chlorkresol ein p-Chlor-m-kresol ist.

7. Verwendung nach einem der Ansprüche 1 bis 6, **dadurch gekennzeichnet, dass** der Desinfektionswirkstoff in einem Anteil von 10 bis 30 Gew.% vorliegt.

8. Verwendung nach einem der Ansprüche 1 bis 7, **dadurch gekennzeichnet, dass** das Desinfektionsmittels mindestens ein Tensid enthält.

9. Verwendung nach Anspruch 8, **dadurch gekennzeichnet, dass** das Tensid in einem Anteil von 10 bis 35 Gew.% enthalten ist.

10. Verwendung nach einem der Ansprüche 1 bis 9, **dadurch gekennzeichnet, dass** das Desinfektionsmittel eine alkalisch wirkende Lauge enthält.

11. Verwendung nach Anspruch 10, **dadurch gekennzeichnet, dass** die alkalisch wirkende Lauge Natronlauge ist.

12. Verwendung nach Anspruch 10 oder 11, **dadurch gekennzeichnet, dass** die alkalisch wirkende Lauge in einem Anteil von 1 bis 15 Gew.%, bezogen auf das Gewicht des Desinfektionsmittels, vorliegt.

13. Verwendung nach einem der Ansprüche 1 bis 9, **dadurch gekennzeichnet, dass** das Desinfektionsmittel eine Säure enthält.

14. Verwendung des Desinfektionsmittels nach einem der Ansprüche 1-13 in einer wäßrigen verdünnten Lösung in einer Konzentration von 0,5 bis 10 Gew.%, bezogen auf die Masse der eingesetzten Lösung.

## Claims

1. Use of a disinfectant agent with an antiparasitic activity, which contains at least one active disinfectant principle and at least one keratolytically active compound, for the non-medical control of ascaridae, coccidia and their permanent forms capable of invasion, protozoa, ticks, lice, sarcoptic mites and microorganisms, the keratolytically active compound being an ester of an organic or inorganic keratolytically active acid and of a monohydric or polyhydric alcohol with 1 to 6 carbon atoms.

2. Use according to claim 1 **characterised in that** the organic acid is thioglycolic acid.

3. Use according to claim 1 or 2 **characterised in that** the ester is a glycerine ester.

4. Use according to claim 1 to 3 **characterised in that** the ester is present in a proportion of 10 to 30% by weight.

5. Use according to one of claims 1 to 4 **characterised in that** the active disinfectant principles are phenolic active disinfectant principles, in particular chlorocresols.

6. Use according to claim 5 **characterised in that** the chlorocresol is a p-chloro-m-cresol.

7. Use according to one of claims 1 to 6 **characterised in that** the active disinfectant principle is present in a proportion of 10 to 30% by weight.

8. Use according to one of claims 1 to 7 **characterised in that** the disinfectant agent contains at least one surfactant.

9. Use according to claim 8 **characterised in that** the surfactant is contained in a proportion of 10 to 35% by weight.

10. Use according to one of claims 1 to 9 **characterised in that** the disinfectant agent contains a lye with an alkaline effect.

11. Use according to claim 10 **characterised in that** the lye with an alkaline effect is caustic soda solution.

12. Use according to claim 10 or 11 **characterised in that** the lye with an alkaline effect is present in a proportion of 1 to 15% by weight, based on the weight of the disinfectant agent.

13. Use according to one of claims 1 to 9 **characterised in that** the disinfectant agent contains an acid.

14. Use of the disinfectant agent according to one of claims 1 to 13 in an aqueous dilute solution in a concentration of 0.5 to 10% by weight, based on the mass of the solution employed.

## Revendications

1. Utilisation d'un agent de désinfection avec activité antiparasitaire, qui contient au moins une substance active de désinfection et un composé agissant de manière kératolytique, pour la lutte non médicinale contre les ascarides, les coccidies ainsi que leur formes durables d'infestation, les protozoaires, les tiques, les poux, les acariens et les microorganismes, où le composé agissant de manière kératolytique est un ester d'un acide organique ou inorganique agissant de manière kératolytique et d'un alcool mono- ou polyvalent avec 1 à 6 atomes de carbone.

2. Utilisation selon la revendication 1, **caractérisée en ce que** l'acide organique est l'acide thioglycolique.

3. Utilisation selon la revendication 1 ou 2, **caractérisée en ce que** l'ester est un ester de glycérine.

4. Utilisation selon l'une quelconque des revendications 1 à 3, **caractérisée en ce que** l'ester est présent en une quantité de 10 à 30% en poids.

5. Utilisation selon l'une quelconque des revendications 1 à 4, **caractérisée en ce que** les substances actives de désinfection sont des substances actives de désinfection phénoliques, en particulier des chlorocrésols.

6. Utilisation selon la revendication 5, **caractérisée en ce que** le chlorocrésol est le p-chloro-m-crésol.

7. Utilisation selon l'une quelconque des revendications 1 à 6, **caractérisée en ce que** la substance active de désinfection est présente en une quantité de 10 à 30% en poids.

8. Utilisation selon l'une quelconque des revendications 1 à 7, **caractérisée en ce que** l'agent de désinfection contient au moins un tensioactif.

9. Utilisation selon la revendication 8, **caractérisée en ce que** le tensioactif est présent en une quantité de 10 à 35% en poids.

10. Utilisation selon l'une quelconque des revendications 1 à 9, **caractérisée en ce que** l'agent de désinfection contient une lessive alcaline.

11. Utilisation selon la revendication 10, **caractérisée en ce que** la lessive alcaline est la soude caustique.

12. Utilisation selon la revendication 10 ou 11, **caractérisée en ce que** la lessive alcaline est présente en une quantité de 1 à 15% en poids, sur base du poids de l'agent de désinfection.

13. Utilisation selon l'une quelconque des revendications 1 à 9, **caractérisée en ce que** l'agent de désinfection contient un acide.

14. Utilisation de l'agent de désinfection selon l'une quelconque des revendications 1 à 13, dans une solution aqueuse diluée en une concentration allant ²de 0,5 à 10% en poids, sur base de la masse de la solution mise en oeuvre.
